# EUROPEAN PATENT SPECIFICATION

(11) **EP 1 528 916 B1**
(45) Date of publication and mention of the grant of the patent: **26.12.2012**
(21) Application number: 03740843.2
(22) Date of filing: 24.07.2003
(51) Int. Cl.: A61K 9/20, A61K 9/48, A61J 3/07

(54) **MULTICOMPONENT PHARMACEUTICAL DOSAGE FORM**
MULTIKOMPONENT PHARMAZEUTISCHE DOSIERFORM
FORME POSOLOGIQUE PHARMACEUTIQUE A MULTICOMPOSANTS

(30) Priority: 25.07.2002 GB 0217336; 03.02.2003 GB 0302435
(43) Date of publication of application: 11.05.2005
(60) Divisional application: 12191988.0
(73) Proprietor: Capsugel Belgium NV, 2880 Bornem (BE)
(72) Inventor: BONNEY, Stanley George, Ware, Hertfordshire SG12 0DP (GB); BROWN, Adrian, GlaxoSmithKline, Harlow, Essex CM19 5AW (GB); DAVIES, Michael Birsha, GlaxoSmithKline, Ware, Hertfordshire SG12 0DP (GB); MARGETSON, Daniel N, GlaxoSmithKline, Harlow, Essex CM19 5AW (GB); MATTHEWS, Wayne M., GlaxoSmithKline, Harlow, Essex CM19 5AW (GB); MCALLISTER, Stephen Mark, GlaxoSmithKline, Harlow, Essex CM19 5AW (GB); RAND, Paul Kenneth, GlaxoSmithKline, Ware, Hertfordshire SG12 0DP (GB); WILSON, Alan Anthony, GlaxoSmithKline, Ware, Hertfordshire SG12 0DP (GB)
(74) Representative: Hyden, Martin Douglas
(86) International application number: PCT/GB2003/003157
(87) International publication number: WO 2004/010978

(56) References cited:
- WO-A-01/08666
- WO-A2-02/096347
- GB-A- 2 148 841
- US-A- 3 186 910
- US-A- 4 673 086
- US-A- 5 074 426
- US-A- 5 674 530

## Description

The present invention relates to a pharmaceutical dosage form, being a multicomponent dosage form comprising components in the form of either a body and a film means, or a capsule shell, linker and a film means, both forms being suitable for oral dosing.

Various types of pharmaceutical dosage form are known for oral dosing. Pharmaceutical capsules are well known, generally being intended for oral dosing. Such capsules generally comprise an envelope wall of a pharmaceutically acceptable, e.g. orally ingestible, polymer material such as gelatin, although other materials for capsule walls, e.g. starch and cellulose based polymers are also known. Such capsules generally have soft walls made by making a film on a capsule former, which is then allowed to dry. Rigid walled capsules made by injection moulding are also known, see for example US 4576284, US 4591475, US 4655840, US 4738724, US 4738817 and US 4790881 (all to Warner Lambert). These disclose specific constructions of capsules made of gelatin, starch and other polymers, and methods of making them by injection moulding of hydrophilic polymer - water mixtures. US 4576284 specifically discloses such capsules provided with a cap which closes the capsule, and which is formed in situ on the filled capsule by moulding. US 4738724 discloses a wide range of rigid capsule shapes and parts. GB 2148841 and US 5074426 relates to a divisible hard shell capsule in dosage form such that the capsule may be divided into subunits. US 5674530 relates a method for making a drug delivery system including the steps of filing a first water permeable capsule half with a drug and an osmotic agent and plugging an open-end of the capsule. US 3186910 relates a method for producing peroral capsules. WO 02/096347 relate to metering and packaging of controlled release medication.

Multi-compartment capsules, including those of the type where each compartment has different drug release characteristics or for example contains a different drug substance or formulation are also known, for example in US 4738724 (Warner-Lambert), US 5672359 (University of Kentucky), US 5443461 (AlzaCorp.), WO 9516438 (Cortecs Ltd.), WO 90/12567 (Helminthology Inst.), DE-A-3727894, BE 900950 (Warner Lambert), FR 2524311, NL 7610038 (Tapanhony NV), FR 28646 (Pluripharm), US 3228789 (Glassman), US 3186910 (Glassman) among others. US 4738817 discloses a multicompartment capsule with a similar construction to those of US 3228789 and US 3186910, made of a water-plasticised gelatin.

Another type of dosage form is disclosed in WO 01/08666 (SmithKline Beecham) wherein the dosage form comprises two or more capsule shells linked together via a linker unit having a plug part which extends into an open end of a shell.

Whilst the dosage form of WO 01/08666 provides advantages over other prior art dosage forms e.g. greater flexibility in producing a dosage form adapted to a patient's specific requirements, there nevertheless remains a need for alternatives. In particular there remains a need for a dosage form that is of a simple construction is easy to manufacture and that can provide a desired drug-release profile e.g. constituting both immediate and delayed release of a drug substance in a single dosage form. In particular it is an object of this invention to provide a dosage form that is easier to fabricate using ultrasonic welding to connect the components together. In the dosage form of WO 01/08666, to form the ultrasonic weld it is normally necessary to transmit ultrasonic energy along the whole length of the capsule shell to weld it to the linker. This can result in problems of shattering of the capsule shell, the need for a high power ultrasonic horn and handling capacity. It is an object of the invention to provide a dosage form that meets these requirements.

According to the invention there is provided a multicomponent pharmaceutical dosage form suitable for retaining drug substance which drug substance can be released to provide a controlled drug release profile in the gastrointestinal environment, characterised by a body having at least one cavity, said cavity having a mouth opening, and a film means connected to the body and closing the mouth opening.

By "controlled drug release" is meant that drug may be released from the dosage form to provide a defined, pre-determined drug release profile. Drug may be released from the dosage form at different rates, at different times following administration to the patient, or in different sites within the patient's gastrointestinal system. For example the dosage form may be designed to provide rapid release, immediate, modified release such as sustained or pulsatile release characteristics e.g. an immediate first pulse of drug followed by a delayed second pulse of drug at some later time point. Preferably the dosage form provides a pulsed release profile resulting in immediate release of a portion of the drug substance contained therein, typically within up to 15 minutes following ingestion of the dosage form, followed by a subsequent or delayed release of another portion of drug contained in the same dosage form, effected at some later point, typically up to 4 hours later. Usually immediate release takes place in the region of the stomach whilst delayed release occurs in another part of the gastro-intestinal compartment such as the small intestine or the gut.

Suitably the dosage form opens to release drug substance in the gastrointestinal environment. The process by which the dosage form opens may comprise one or more physical and/or chemical processes. For example the film means, the body, or a connection between the two such as a weld, may on exposure to a particular gastrointestinal environment and/or after a suitable time delay, shear or burst to release the content of a part or all of the dosage form. For example welded parts may separate as a consequence of differential swelling on hydration of the parts. Alternatively a component of the dosage form e.g. the film means, the body, a connection between the two, may dissolve partly or wholly or be otherwise breached to release the content of part or all of the dosage form.

Suitably the invention may comprise a plurality of cavities and/or a plurality of film means.

The present invention relates to a multi-component pharmaceutical dosage form suitable for retaining drug substance which drug substance can be released to provide a controlled drug release profile in the gastrointestinal environment, characterized by a body comprising a base wall having an upper surface, a first cavity is defined by a skirt wall extending away from the upper surface of the said base wall to terminate in a rim defining a first mouth opening, a second cavity is defined by a skirt wall extending away from the lower surface of said base wall to terminate in a rim defining a second mouth opening, and a first and second film means connected to the body closes the first and second mouth openings;wherein the body and/or the film means is made of materials selected from a polymer which dissolves or disintegrates in the environment of the upper or lower digestive tract, or which hydrates in the digestive tract, such that the dosage form opens to release the drug substance in the gastro intestinal environment, and wherein the film means is connected to the body by a weld.

Suitably in this embodiment the body may be shaped so as to define a first and second cavity therein. More particularly the body may comprise a base wall having an upper and a lower surface, the first cavity being defined by a first skirt wall extending upwardly (hereinafter referred to as an 'upward' direction) away from the upper surface of the base wall to terminate in a rim defining the first mouth opening, the second cavity being defined by a second skirt wall extending downwardly (hereinafter referred to as a 'downward' direction) away from the lower surface of the base wall to terminate in a rim defining the second mouth opening. In such an embodiment the body may be generally 'H' shaped in longitudinal section with the first and second cavities formed in the two opposing hollows between the horizontal linker portion of the H' and the side arms of the 'FT. By "generally 'H"' shaped is also intended to encompass other like shaped body configurations that give rise to a cavity or cavities either side of a base wall e.g. a body comprising a flattened 'H' shape in section wherein the length of the arms of the 'FT is equal to or shorter than the width of the linker portion between the arms. The term 'H' shaped includes shapes with parallel, curved, convergent or divergent arms. Suitably the ratio of the length of the skirt walls: width of the base wall is in the range 3: 1 to 1 :5. Suitably the first and second film means are connected to an upper and lower rim, respectively, of the skirt walls, to thereby close the first and second mouth openings.

Suitably the film means is connected to the respective rims of each mouth opening and serves to close the mouth openings thereby sealing each cavity enabling drug substance and any other desired material, e.g. a pharmaceutically acceptable carrier, to be retained therein. The first and/or second film means may form a blister convex relative to the respective first and/or second cavity. The first/and or second film means may form a substantially planar surface relative to the first and/or second cavity. Preferably the film means is substantially planar and may be connected to a correspondingly substantially planar portion of the body, e.g. of the rim of the skirt wall, thereby facilitating a good seal between the component parts.

In this embodiment the body may be made of an inert polymer, i.e. which does not dissolve in the gastro intestinal (GI) environment e.g. which passes through the GI tract. Preferably the body is made of a delayed release polymer which remains substantially intact e.g undissolved until the body has reached the intestine, or a pulsatile release polymer.

The first and second film means may independently comprise an immediate release or delayed or pulsatile release polymer, e.g. both may comprise an immediate release polymer or both may comprise a delayed release polymer, or one may be immediate release and the other delayed release polymer.

Advantageously the invention may provide a dosage form in a desired shape. For example the dosage form may be substantially cylindrical, which includes shapes which have a circular, oval or oblate circular cross section across the longitudinal axis, and shapes which have parallel or tapering e.g. with side walls which taper conically, which includes both true cones i.e. with straight side walls, and cones with curved side walls, over at least part of their extent. Dosage forms that are substantially elongated or circular when viewed in cross section across the longitudinal axis but being short in their longitudinal direction relative to their dimension across the cross section may resemble respectively capsules or tablets. Dosage forms that resemble tablets are preferred, e.g. longitudinally flattened cylinders, flattened spheroids or flattened ellipsoids.

Film means of the invention may be produced in a number of ways e.g. by hot-melt extrusion, solvent casting, or by an injection moulding process to produce, respectively, a continuous sheet / layer, or a series of discrete film parts. Preferably the film means is in the form of a sheet / layer that may be cut to size to fit the corresponding body. Film means may also be combined to form multi-laminated constructions of film means in which different polymer films are joined to create a sandwich-type layer with increased functionality compared to single layer films. Suitably the thickness of the film means is in the range 20-300 µm (micron) preferably 25-100 µm (microns). A body according to the invention may be produced by injection moulding processes which can enable the body to be made with precision. Suitable techniques of injection moulding are known for example from the art of manufacture of small plastic components. Processes such as those described in Cuff. G and Raouf. F, Pharmaceutical Technology, June 1998, p96-106, may be used to manufacture body parts. Consequently the invention also provides a moulding process for example an injection moulding or powder compression process wherein the body part of the dosage form is made in a mould cavity. Preferably the moulding process is a hot runner process. The invention also provides a mould or die, suitable for use in the moulding process. Such a mould or die may have a mould cavity corresponding to the shape of the body. Moulds may be made by known metal engraving processes such as spark erosion and it is generally preferred to use moulds made from pharmaceutically acceptable metals e.g. steels of the type known for use in tablet compression dies.

In an assembled dosage form of the invention, the component parts of the dosage form namely the film means and the body may be connected together e.g. by a weld such as a thermal weld, an ultrasonic weld, inductive weld or an adhesive weld (e.g. curable adhesives such as UV curable adhesive). A thermal weld may for example be achieved by bringing the film means and the body into adjacent contact and applying localised heating for example produced by directing a laser beam or a fine jet of hot gas e.g. nitrogen at the area where the film means and the body are in contact. In thermal, inductive and ultrasonic welding normally localised fusion together of the materials of adjacent parts of the dosage form which are in contact occurs, and on subsequent solidification of the materials a bond is formed between the adjacent parts. An adhesive weld may be achieved by applying an adhesive to at least one part of the dosage form which when the dosage form is assembled is in contact with another part, and then causing or allowing the adhesive to set.

The multicomponent dosage form of the present invention is particularly suited to fabrication using ultrasonic welding. Ultrasonic welding is a known technique involving the use of high frequency sound energy to soften or melt a thermoplastic material at the site where a joint with the material is required. A general description of ultrasonic welding is for example to be found in the publication "Ultrasonic Welding of Thermoplastics" (TWI Ltd., Abington, Cambridgeshire GB, (1997)). Parts to be joined are held together under pressure and then subjected to ultrasonic vibrations usually at a frequency of 20 - 40 kHz. The actual mechanism responsible for the generation of heat at the joint site is not well understood. An ultrasonic welding machine comprises five main components, being a power supply, a control system, a welding head, fixturing to hold the parts to be welded, and a system to apply the required pressure. The power supply converts electricity into high frequency electric power which drives a transducer, e.g. a piezoelectric transducer, which converts electrical energy, e.g. from the mains supply, into mechanical, i.e. ultrasonic, energy. Between the transducer and the parts to be welded is located a booster and horn system, being a usually metallic component which serves to amplify the ultrasonic waves (the booster horn), transmit the clamping pressure, and deliver the sound energy to the part to be welded (the sonotrode or welding horn). For successful ultrasonic welding careful design of the parts to be welded and set up of the welding equipment is important. The rim of the mouth opening may be profiled to facilitate formation of an ultrasonic weld.

It will be apparent to those skilled in the art how a mouth opening of a cavity may be made of a suitable size and profile to facilitate the welding of a film means to the rim to close the mouth opening. One suitable profile of the rim of the mouth opening to facilitate ultrasonic welding of the film means thereto may comprise a rim in the form of an upwardly facing generally flat surface generally perpendicular to the depth direction of the cavity. Optionally such a surface may be bounded by a kerb edge e.g. a small upwardly extending wall around the outer periphery of the rim. The film means may be placed on top of such a surface with one side of the film in contact with the surface, and by applying the ultrasonic horn to the film means on the opposite side of the film, the weld may be formed. There may be one or more small upwardly extending ridges on the surface which may collapse on application of the horn to the weld. Additionally or alternatively the rim may have a concavity such as a groove therein into which the film means e.g. an edge thereof, may fit.

Alternatively the rim may be flat and the ultrasonic horn may have one or more small upwardly extending ridges on the surface acting on the film in order to focus sound energy on a specific point or points to achieve welding of the two components. It is preferred to use an ultrasonic horn which can both form the weld and cut the film means to a suitable size and shape corresponding to the mouth opening and its rim.

The film means and body of a dosage form of the invention are made of materials having particular physico-chemical characteristics to achieve a desired release profile. The film means and the body may be comprised of material designed to provide rapid dissolution, immediate dissolution and/or modified dissolution e.g. delayed dissolution to confer sustained or pulsatile release characteristics, and combinations thereof. For example in an embodiment according to the invention e.g. in the first embodiment herein described, the first film means may be designed to provide rapid or immediate dissolution and the second film means may provide modified dissolution such as sustained or pulsatile release characteristics. In an alternative arrangement of the first embodiment the first film means may be designed to provide rapid or immediate dissolution and the body may be designed to provide modified dissolution. In an alternative embodiment, such as a variant of the second embodiment, the film means may be designed to provide modified dissolution and the body, e.g. in the vicinity of the weakened portion may provide rapid or immediate dissolution.

Suitable materials for the provision of a desired release profile include pharmaceutically acceptable polymers and blends thereof which may be injection moulded to form a body such as a capsule shell or linker, and may also be made into thin films to provide the film means of the dosage form. If the component parts of the dosage form, e.g. body and film means are to be welded together e.g. by an ultrasonic weld then preferably polymers or blends thereof are selected for the respective parts to be welded which are compatible to encourage weld formation. For example the polymer of each of such components may be blended with a common polymer to facilitate this.

For purposes herein representative examples of polymers suitable for injection molding or film-formation into multicomponent dosage forms and for use in pharmaceutical applications, include, but are not limited to, poly(ethylene) oxides (PEO), polyethylene glycol's (PEG), mixtures of PEG's and PEO's, polyvinyl alcohol (PNA), polyvinyl acetate, povidone (polyvinyl pyrrolidone), cellulose derivatives such as carboxymethyl cellulose, methyl cellulose, ethylcellulose, hydroxyethyl cellulose, hydroxypropylcellulose, hydroxyethyl mefhylcellulose, hydroxy propylmethyl cellulose (HPMC) e.g. those sold under the tradename Klucel, , hydroxypropylmefhyl cellulose phthalate, cellulose acetate phthalate, noncrystalline cellulose, starch and its derivatives such as hydroxyethyl starch, sodium starch glycolate, natural polymers (such as polysaccharides like pullulan, carrageenan, xanthan, chitosan or agar gums), polyacrylates and poly (meth)acrylates, and its derivatives such as the Eudragit family of polymers available from Roehm Pharma, poly(alpha-hydroxy acids) and its copolymers such poly(caprolactone), poly(lactide-co-glycolide), poly(alpha-aminoacids) and its copolymers, polyglycolysed glycerides (such as Gelucire® 44/14, Gelucire® 50/02, Gelucire® 50/13 and Gelucire® 53/10), carboxyvinyl polymers (such as Carbopols), and polyoxyethylene-polyoxypropylene copolymers (such as Poloxamer 188™); and combinations or mixtures thereof.

Also potentially suitable for use herein are the polymers poly(orthoesters), polyphosphazenes, poly(phosphoesters), and polyanhydrides, and combinations or mixtures thereof.

Additionally, hyaluronic acid, alginates, carragenen, collagen, gelatin, and albumen may also be suitable for injection moulding herein, either alone or in combination with another polymeric blend. It is recognised that the ultimate choice of polymers if not previously approved by the regulatory agencies of the world, are in the category of generally recognised as safe (GRAS) approved.

Especially suitable polymers for use in the invention are those that preferentially dissolve or disintegrate at a defined point or region in the digestive tract.

For example a polymer may dissolve or disintegrate in the environment of the upper digestive tract or the stomach, and so be termed an "immediate" release polymer, for example dissolving or disintegrating within ca. 1 hour, e.g. within ca. 30 minutes of oral administration. For example a polymer may dissolve or disintegrate in the environment of the lower digestive tract e.g. the intestine, and so be termed a "delayed" or "pulsed" release polymer, for example substantially not releasing any medicament contained therein until the polymer has reached the intestine. Such polymers include the known acrylic and/or methacrylic acid-based polymers which are soluble in intestinal fluids e.g. the Eudragit series of polymers, produced by Roehm GmbH in Germany. Examples of Eudragit polymers include Eudragit E e.g. Eudragit E100, which preferentially dissolves in an acidic, e.g. up to pH 5, environment e.g. in the acidic environment of the stomach, or enteric polymers such as Eudragit L, e.g. Eudragit L100, and/or Eudragit S, which preferentially dissolve in a more alkaline pH environment e.g. in the environment of the intestine. Suitably the polymer Eudragit 4135F dissolves only above pH7 e.g. in the colon and so is suitable for formulation as a delayed release or pulsatile release component. However this polymer may be formulated so as to be pH independent, as discussed herein below. Other suitable polymers also include polymers which are insoluble but hydrate at a controlled rate e.g. at a predetermined rate in the digestive tract such as Eudragit RL e.g. Eudragit RL 100 and/or Eudragit RS e.g. Eudragit R100 and/or blends of Eudragit polymers. The Eudragit polymers i.e. poly(meth) acrylate copolymers, such as Eudragit 4135F, Eudragit E100 and Eudragit L100 are included as preferred polymers for use in forming the component parts of a dosage form according to the invention namely one or more of a body, a film means, a capsule shell and/or a linker. A particular polymer disclosed in US 5,705,189, Emulsion E2 (column 6 line 10), being a copolymer of methacrylic acid, methyl methacrylate and methyl acrylate, suitably in a ratio of 10:25:65, is a preferred polymer for use in the present invention (as are the polymers disclosed in WO 01/43935 and WO 01/39751, both in the name of Roehm). This ratio of components is also known as Eudragit 4135F and is a solid product obtained from Eudragit FS30D, and as noted above is available from Roehm Pharma, Germany.

Eudragit E100 comprises butylmethacylat-(2-dimethylaminoethyl) methacrylat-methylmethacylat-copolymer (1 :2:1) and is a copolymer based on (2-dimethylaminoethyl)methacrylalate, butyl methacrylate and methylmethacrylate, typically in a 2:1 :1 ratio, having a mean molecular weight of about 150,000. It contains not less than 20.8 and not more than 25.5% dimethylaminoethyl groups in the dry substance. Eudragit L100 comprises polymethacrylic acid, methyl methacrylate) in a ratio of 1 : 1.

Suitably a delayed release or pulsed release body, film means, capsule shell or linker comprises a blend based on Eudragit 4135F, or a blend based on Eudragit L100. Suitably an immediate release body, film means, capsule shell or linker comprises Eudragit E100, RL100, RS 100, hydroxypropylcellulose, or a blend based thereon. A preferred combination of polymers for use in any film means/body combination comprises Eudragit E100 or hydroxypropylcellulose for immediate release, and Eudragit E4135F and/or Eudragit L100 for delayed or pulsed release.

When the delayed release or pulsed release polymer comprises Eudragit 4135F, this polymer is suitably blended with at least one lubricant and at least one dissolution modifying agent to achieve quality, non-distortion moulded components which readily release from injection moulds. Examples of suitable dissolution modifying agents, lubricants and other optional additional additives or excipients are provided below. Suitably Eudragit 4135F is used in an amount of about 20 to about 90% w/w , preferably from about 50 to 90%w/w, the dissolution modifying excipient is used in an amount of about 2.5 to about 30% w/w, and the lubricant in an amount of about 5 to about 30%w/w. A suitable polymer blend comprises a polymer e.g. Eudragit 4135F ca. 78%w/w, a lubricant e.g. stearyl alcohol ca. 12%w/w, and a dissolution modifying agent e.g. hydroxypropylmethylcellulose ca.10%w/w. Suitable blends based on 4135F are for example disclosed in WO 02/06038. When the delayed release or pulsed release polymer comprises Eudragit L100, the polymer is preferably blended with at least one lubricant and at least one plasticizer. Suitably the polymer is used in an amount of about 40 to about 70%w/w, the lubricant is used in an amount of about 2 to about 10%, and the plasticizer is used in an amount of about 20 to about 50% w/w. A suitable polymer blend comprises a lubricant e.g. glyceryl monostearate ca. 5%, a plasticizer e.g. triefhyl citrate ca.40% and Eudragit LI 00 up to 100%w/w.

Alternatively the delayed release polymer may comprise a graft copolymer comprising PVA and PEG. One commercially available form of such a polymer comprises 75% PNA and 25% PEG and is available under the trade name Kollicoat IR.

When the immediate release polymer comprises Eudragit E100, the polymer may be used either by itself or as a blend. Suitably a blend may comprise one or more dissolution modifying agent(s) and one or more lubricant(s). Suitably the polymer is used in an amount of about 30 to about 90% w/w, the dissolution modifying excipient is used in an amount of about 0 to about 70%w/w e.g. in the range 5 to 70%w/w, and the lubricant is used in an amount up to about 30%w/w. Preferably the polymeric blend further comprises a plasticiser in the range from 0% to about 5% and a processing agent in the range 0 to 30%w/w. A suitable polymer blend comprises a polymer e.g. Eudragit E100 ca 53%, a lubricant e.g. stearyl alcohol ca. 12%, a processing aid e.g. polyethylene oxide ca. 20%, a strengthening aid e.g. talc ca. 10% and co-povidone ca. 5%. Suitable blends are for example disclosed in WO 02/060385.

An immediate release polymer may comprise a hydroxyethyl cellulose, low molecular weight hydroxypropylcellulose and/or a low-substituted hydroxypropyl cellulose. Further, two or more polymers may be used in combination to form blends having the desired characteristics. For example it is known from WO02/060384 that a novel blending of components has the ability to render the poly(methacrylates), such as Eudragit 4135F, which are pH dependent independent of this characteristic. A suitable immediate release or pulsed release blend may comprise one or more cellulose derivatives in an amount from about 20-80%w/w e.g. hydroxypropylcellulose derivatives of varying molecular weights available commercially as Klucel e.g. Klucel EF and Klucel JF, having a molecular weight respectively of ca. 80000 and ca. 140000, each being present in an amount ca. 32%, 5-30% of a lubricant e.g. stearyl alcohol ca. 12%w/w, and 20-90%w/w of a polymer e.g. ca. 23%o Eudragit 4135F. Preferably such a blend is used in a capsule shell according to the invention. When welded to a delayed release component based on Eudragit 4135F as described above, such a weld may separate as a result of differential swelling on hydration.

It is found that a 20-300 µm (micron) thick film of the above-mentioned immediate release polymers can dissolve in less than 5 minutes in the environment of the digestive tract. However if a film based on one or more such immediate release polymers is to be welded e.g. ultrasonically to a body made of a different polymer such as a polymer or polymer blend containing a Eudragit polymer, it is preferable to blend the polymer of the film with a polymer included in the body it is to be welded to.

The aforementioned polymer-based body/ film means combinations, and capsule shell/linker combinations may be used in any one of the dosage forms according to the embodiments of the invention. For example a dosage form according to the first embodiment may comprise a body comprising Eudragit 4135F and a film means comprising Eudragit E100 and/or Eudragit L100. For example the body may comprise Eudragit 4135F and a first and second film means may comprise Eudragit E100 or Eudragit L100 respectively, or alternatively a first film means may comprise Eudragit E100 and a second film means may comprise Eudragit L100.

In general, for use herein, suitable dissolution modifying excipients include a disintegrant such as sodium starch glycollate e.g. Explotab, cross-linked PNP e.g. Kollidon-CL, copovidone e.g. KoUidon VA 64 or starch e.g. starch 1500; a swelling agent such as polyvinylpyrrolidone (PVP, also known as povidone) e.g. ISP-plasdone or BASF-Kollidon and primarily grades with lower K values e.g. K-15, K25 and K-30; a cellulosic derivative such as hydroxypropylme hylcellulose; a wicking agent such as a low molecular weight solute e.g. mannitol, lactose and starch; inorganic salts such as sodium chloride (typically at 5-10%). Suitable lubricants or glidants include stearyl alcohol, stearic acid, glycerol monostearate (GMS), talc, magnesium stearate, silicon dioxide, amorphous silicic acid, fumed silica and combinations thereof. Suitable plasticisers include triethyl citrate (TEC), triacetin, tributyl citrate, acetyl triethyl citrate (ATEC), acetyl tributyl citrate (ATBC), dibutyl phthalate, dibutyl sebacate (DBS), diethyl phthalate, vinyl pyrrolidone glycol triacetate, polyethylene glycol, polyoxyethylene sorbitan monolaurate, propylene glycol, or castor oil; and combinations or mixtures thereof. The choice of polymer will determine which plasticiser is suitable for use and this will be apparent to the man skilled in the art. For instance, triacetin is not preferred for use with E100 or 4135F at levels of about 5% but may be suitable for use with Eudragit RS or RL, or PVA.

The dosage form of the invention may be wholly or partly coated with a polymeric coating, such as the Opadry film coating system, e.g. to influence its release characteristics and/or to protect an otherwise fragile film means and/or to modify the appearance of the dosage form. A coating may comprise a delayed or pulsed release polymer which dissolves or is otherwise be breached in an environment of defined pH, such as that of the stomach or intestine, such as the Eudragit polymers discussed above. A typical such polymer is the enteric polymer Eudragit L30D 5S which dissolves in the intestine. For example a capsule shell made of an immediate-release polymer as described above may be coated with such a coating. Processes are well known in the art for applying such a coating to a substrate, and which are applicable to a component of a dosage form. Generally it may be preferable to apply such a coating to the components of the dosage form, e.g. the first or second body, capsule shells etc. before filling with a drug substance, because typical processes for applying such coatings involve heating the substrate to an elevated temperature, which may be detrimental to a drug substance contained therein.

The dosage form is particularly suitable for presentation as an oral dosage form containing one or more drug substances e.g. containing a combination of drug substances. When there is more than one cavity present each cavity may contain the same or different drug substance which may be released at the same time or a different rate or time after administration or place in the patient's gastro-intestinal system. Additionally or alternatively each of two or more cavities may contain respective substances which are incompatible in contact with each other prior to administration to a patient. Advantageously the invention provides a dosage form having variable drug content and/or drug release characteristics to provide a dosage form tailored to specific administration requirements. The drug substance(s) contained in the cavity(ies) may be present in any suitable form e.g. in the form of powder, pellets, granules, semisolids or liquids.

Dosage forms of the invention may be prepared by various processes. A typical process for making a dosage form comprising two cavities comprises the following steps:
1. Forming a body e.g. by injection moulding of a suitable polymer and forming a film means e.g. by hot-melt extrusion.
2. Optionally applying a polymer coat to the body.
3. Filling a first or second cavity with drug substance.
4. Closing the so-filled cavity with a film means.
5. Filling a second or first cavity with the same or different drug substance.
6. Closing the so-filled cavity.

When the dosage form comprises a body having two oppositely facing i.e. upwardly and downwardly facing mouth openings it is preferred to perform steps 3 and 4 on the cavity which is facing upwardly then to invert the body so the other cavity is facing upwards, then perform steps 5 and 6.

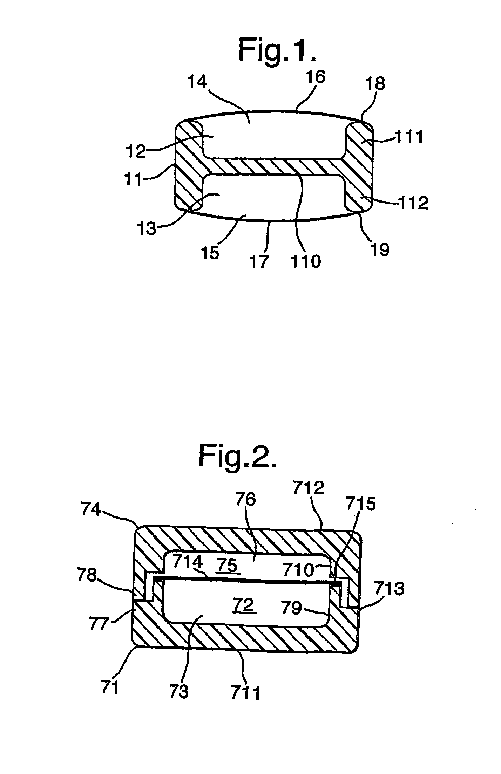

In an alternative aspect of the invention there is also provided the component parts of the invention e.g. a body for use in a dosage form according to the invention and comprising at least one cavity having a mouth opening and adapted for connecting a polymer film thereto. Details of the dosage forms referred to above will now be described with reference to Figs.1-12 which show longitudinal plans and perpendicular views of a dosage form of the invention and/or components thereof.

Referring to Fig. 1 a dosage form is shown having an body 11, H' -shaped in longitudinal section comprising a first cavity 12 and a second cavity 13, the first and second cavities having a first and second mouth opening, 14 and 15, respectively. The body 11 is made of a delayed dissolution polymer, by injection moulding. The first and second mouth openings 14 and 15 are closed by first and second film means 16 and 17 respectively connected by an ultrasonic weld to the respective rim 18, 19 of the mouth opening 14, 15. The first and second film means 16 and 17 form a blister convex relative to the respective first and second cavity. The first film means 16 may comprise material that confers delayed release characteristics upon the dosage form e.g. Eudragit 4135F, whilst the second film means 17 may comprise material that confers immediate release e.g. Eudragit E100. The body comprises a base wall 110 and skirt walls 111,112 extending respectively upwardly and downwardly from the base wall 110 to define the cavities 12,13. The cavities 12 and 13 contain drug substance (not shown) and, if desired, any pharmaceutically acceptable carrier.

Referring to Fig. 2 a dosage form is shown comprising a first body 71 having a first cavity 72 having a mouth opening 73 and a second body 74 comprising a second cavity 75 having a mouth opening 76. The bodies 71, 74 are connected to each other around the respective rims 77 and 78 of skirt walls 79 and 710, extending respectively upwardly and downwardly from base walls 711, 712, of the first and second bodies 71 and 74 at weld 713. A film means 714 is welded at 715 to skirt wall 75 of body 71 to close mouth opening 73. Each cavity 72 and 75 contain drug substance (not shown). The first body 71 may comprise immediate release material and the second body 74 and the film means 714 may comprise delayed release material.

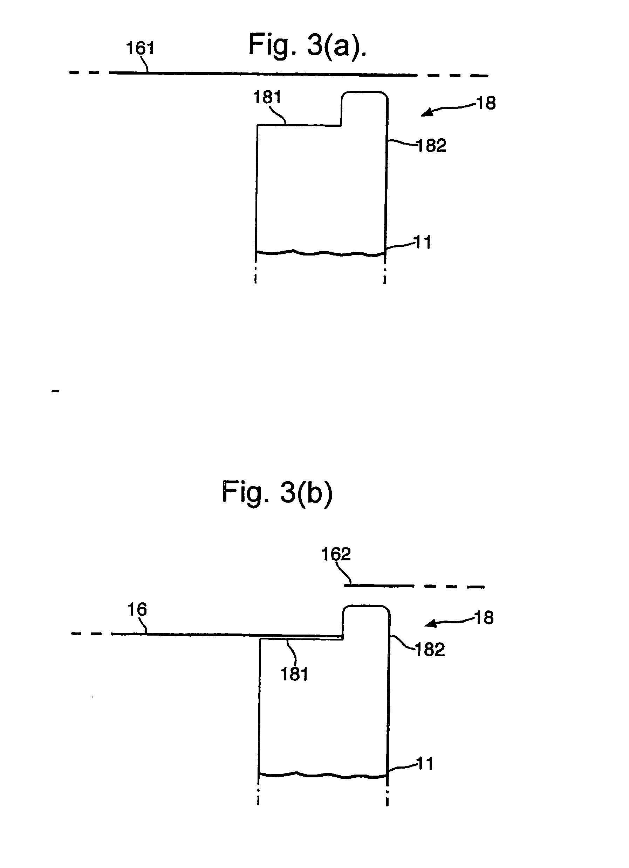

Fig. 3A and Fig 3B, show a construction of the rim 18 of a dosage form according to Fig.1, suitable for forming an ultrasonic weld. The rim 18 comprises a generally flat upwardly facing surface region 181, bounded at its outer periphery by a small upstanding kerb 182. A small upstanding ridge (not shown) may be provided on flat surface 181 to facilitate formation of the weld. A film means 161 is welded to the surface 181 by positioning a sheet 161 of the film material above the rim 18 as shown in Fig. 8A, then bringing down an ultrasonic horn (not shown) on to the sheet 161 to compress and weld the sheet 161 material against surface 181, and simultaneously to cut the sheet material 161 at edge 162. As shown in Fig 8B the film means 16 is thereby connected by a weld to the body 11 and by the kerb 182.

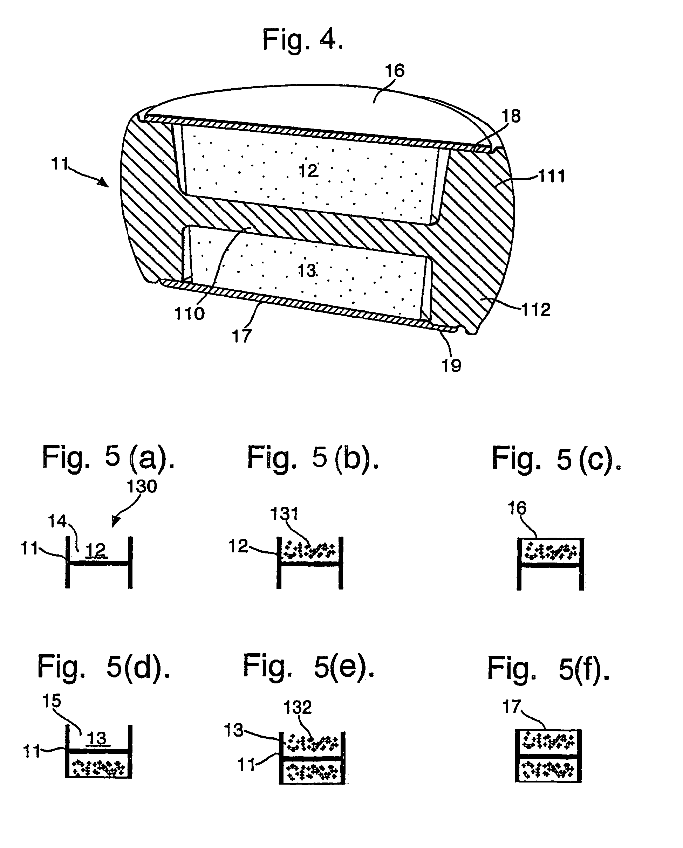

Fig. 4 shows a perspective view of a dosage form according to Fig.1.

Fig. 5 shows how a dosage form 130 according to the first embodiment, i.e. Figs. 1, 3 and 4 may be filled. As seen in Fig. 5A a body 11 is provided, oriented so that its first cavity 12 with its mouth opening 14 is facing upwardly. As seen in Fig. 5B drug substance 131 is introduced into cavity 12 by a suitable filling means (not shown). As seen in Fig 5C the mouth opening 14 is closed by a film means 16 ultrasonically welded thereto in a manner as described above. As seen in Fig. 5D the dosage form is inverted so that the body 11 is oriented so that its second cavity 13 with its mouth opening 15 is facing upwardly. As seen in Fig. 5E drug substance 132 is introduced into cavity 13 by a suitable filling means (not shown). As seen in Fig 5F the mouth opening 15 is closed by a film means 17 ultrasonically welded thereto in a manner as described above. The so-made dosage form 130 may now be further processed e.g. coated.

## Claims

1. A multi-component pharmaceutical dosage form suitable for retaining drug substance which drug substance can be released to provide a controlled drug release profile in the gastrointestinal environment, **characterized by**:
a body (11) comprising a base wall (110) having an upper surface, a first cavity (12) is defined by a skirt wall (111) extending away from the upper surface of the said base wall (110) to terminate in a rim (18) defining a first mouth opening (14), a second cavity (13) is defined by a skirt wall (112) extending away from the lower surface of said base wall (110) to terminate in a rim (19) defining a second mouth opening (15), and a first and second film means (16, 17) connected to the body (11, 21) closes the first and second mouth openings(14, 15);
wherein the body (11) and/or the film means (16, 17) is made of materials selected from a polymer which dissolves or disintegrates in the environment of the upper or lower digestive tract, or which hydrates in the digestive tract, such that the dosage form opens to release the drug substance in the gastro intestinal environment, and wherein the film means (16, 17) is connected to the body (11) by a weld.

2. A multi-component pharmaceutical dosage form according to claim 1 **characterized in that** the weld is an ultrasonic weld.

3. A dosage form according to claim 1 **characterized in that** the film means is hydroxyethyl cellulose or low-substituded hydroxypropyl cellulose.

4. A dosage form according to claim 1, comprising said body (11), base wall (110), first cavity (12) and second cavity (13) and said first and second film means (16, 17) closing said first and second mouth openings (14, 15), **characterized in that** the first film means (16) provide rapid or immediate dissolution, and said second film means (17) provides sustained or pulsatile release.

5. A process for the preparation of a dosage form according to any one of claims 1 to 4, **characterized by** the following steps:
1. Forming a body (11)
2. Optionally applying a polymer coat to the body.
3. Filling a first cavity (12) with drug substance 20
4. Closing the first cavity (12) with a film means
5. Filling a second cavity (13) with the same or different drug substance
6. Closing the second cavity (13).

## Patentansprüche

1. Pharmazeutische Multikomponenten-Dosierungsform, welche für die Aufnahme einer Arzneimittelsubstanz geeignet ist, wobei die Arzneimittelsubstanz freigesetzt werden kann, um ein kontrolliertes Arzneimittel-Freisetzungsprofil im gastrointestinalen Milieu bereitzustellen, **gekennzeichnet durch**:
einen Körper (11), umfassend eine Basiswand (110) mit einer oberen Oberfläche, eine erste Aussparung (12), die **durch** eine Schürzenwand (111) definiert ist, welche sich von der oberen Oberfläche der genannten Basiswand (110) erstreckt, um in einer Randzone (18) zu enden, welche eine erste Mundöffnung (14) bildet, eine zweite Aussparung (13), die **durch** eine Schürzenwand (112) definiert ist, welche sich von der unteren Oberfläche der genannten Basiswand (110) erstreckt, um in einer Randzone (19) zu enden, die eine zweite Mundöffnung (15) definiert, und eine erste und zweite Folie (16, 17), die mit dem Körper (11, 21) verbunden sind, welche die erste und zweite Mundöffnung (14, 15) schließen,
wobei der Körper (11) und/oder die Folie (16, 17) aus Materialien hergestellt sind/ist, ausgewählt aus einem Polymer, welches sich in dem Milieu des oberen oder unteren Verdauungstraktes auflöst oder zerfällt oder in dem Verdauungstrakt hydratisiert wird, sodass die Dosierungsform sich öffnet und die Arzneimittelsubstanz in dem gastrointestinalen Milieu freigesetzt wird und wobei die Folie (16, 17) mit dem Körper (11) **durch** eine Naht verbunden ist.

2. Pharmazeutische Multikomponenten-Dosierungsform nach Anspruch 1, **dadurch gekennzeichnet, dass** die Naht eine ultraschallgeschweißte Naht ist.

3. Dosierungsform nach Anspruch 1, **dadurch gekennzeichnet, dass** die Folie Hydroxyethylcellulose oder nieder substituierte Hydroxypropylcellulose ist.

4. Dosierungsform nach Anspruch 1, umfassend den Körper (11), die Basiswand (110), die erste Aussparung (12) und die zweite Aussparung (13) und die genannte erste und zweite Folie (16, 17), die die genannte erste und zweite Mundöffnung (14, 15) schließen, **dadurch gekennzeichnet, dass** die erste Folie (16) eine rasche und sofortige Auflösung bereitstellt und die genannte zweite Folie (17) eine anhaltende oder schubweise Freisetzung bereitstellt.

5. Verfahren zur Herstellung einer Dosierungsform nach einem der Ansprüche 1 bis 4, **gekennzeichnet durch** die folgenden Schritte:
1. Bilden eines Körpers (11)
2. Optionales Aufbringen einer Polymerschicht auf den Körper
3. Füllen einer ersten Aussparung (12) mit einer Arzneimittelsubstanz (20)
4. Schließen der ersten Aussparung (12) mit einer Folie
5. Füllen einer zweiten Aussparung (13) mit der gleichen oder einer anderen Arzneimittelsubstanz
6. Schließen der zweiten Aussparung (13)

## Revendications

1. Forme posologique pharmaceutique à multicomposants convenant pour retenir une substance médicamenteuse, laquelle substance médicamenteuse peut être libérée pour assurer un profil de libération de médicament contrôlée dans l'environnement gastro-intestinal, **caractérisée par** :
un corps (11) comprenant une paroi de base (110) ayant une surface supérieure, une première cavité (12) définie par une paroi en collerette (111) s'écartant de la surface supérieure de ladite paroi de base (110) pour se terminer par un rebord (18) définissant une première ouverture (14), une seconde cavité (13) définie par une paroi en collerette (112) s'écartant de la surface inférieure de ladite paroi de base (110) pour se terminer par un rebord (19) définissant une seconde ouverture (15) et un premier et un second moyen de pelliculage (16, 17) raccordés au corps (11, 21) fermant la première et la seconde ouverture (14, 15) ;
dans laquelle le corps (11) et/ou les moyens de pelliculage (16, 17) sont constitués de matières choisies parmi un polymère qui se dissout ou se désintègre dans l'environnement des voies digestives supérieures ou inférieures ou qui s'hydrate dans les voies digestives, de sorte que la forme posologique s'ouvre pour libérer la substance médicamenteuse dans l'environnement gastro-intestinal et dans laquelle les moyens de pelliculage (16, 17) sont raccordés au corps (11) par une soudure.

2. Forme posologique pharmaceutique à multicomposants selon la revendication 1, **caractérisée en ce que** la soudure est une soudure ultrasonique.

3. Forme posologique selon la revendication 1, **caractérisée en ce que** les moyens de pelliculage sont formés d'hydroxyéthylcellulose ou d'hydroxypropylcellulose faiblement substituée.

4. Forme posologique selon la revendication 1, comprenant ledit corps (11), ladite paroi de base (110), ladite première cavité (12) et ladite seconde cavité (13) et lesdits premier et second moyens de pelliculage (16, 17) fermant lesdites première et seconde ouvertures (14, 15), **caractérisée en ce que** le premier moyen de pelliculage (16) assure une dissolution rapide ou immédiate et ledit second moyen de pelliculage (17) offre une libération soutenue ou pulsée.

5. Procédé de préparation d'une forme posologique selon l'une quelconque des revendications 1 à 4, **caractérisé par** les étapes suivantes consistant à :
1. former un corps (11),
2. appliquer éventuellement une couche de polymère au corps,
3. remplir une première cavité (12) par une substance médicamenteuse (20),
4. fermer la première cavité (12) par un moyen de pelliculage,
5. remplir une seconde cavité (13) avec une substance médicamenteuse identique ou différente, et
6. fermer la seconde cavité (13).
